# FASCICULE DE BREVET EUROPEEN

(11) **EP 3 119 867 B1**
(45) Date de publication et mention de la délivrance du brevet: **06.05.2020**
(21) Numéro de dépôt: 14721434.0
(22) Date de dépôt: 21.03.2014
(51) Int. Cl.: C12F 3/06

(54) **UTILISATION DE RAFLES LIGNIFIEES OU D'AU MOINS UN EXTRAIT DE RAFLES LIGNIFIEES DANS UNE COMPOSITION COSMETIQUE, UN COMPLEMENT ALIMENTAIRE, UN PRODUIT NUTRITIONNEL, UN ALIMENT OU UNE BOISSON**
VERWENDUNG VON VERHOLZTEN STÄNGELN ODER VON MINDESTENS EINEM EXTRAKT AUS VERHOLZTEN STÄNGELN IN EINER KOSMETISCHEN ZUSAMMENSETZUNG, EINEM NAHRUNGSERGÄNZUNGSMITTEL, EINEM ERNÄHRUNGSPRODUKT, EINEM LEBENSMITTEL ODER EINEM GETRÄNK
USE OF LIGNIFIED STALKS OR OF AT LEAST ONE EXTRACT OF LIGNIFIED STALKS IN A COSMETIC COMPOSITION, A FOOD SUPPLEMENT, A NUTRITIONAL PRODUCT, A FOOD OR A BEVERAGE

(43) Date de publication de la demande: 25.01.2017
(73) Titulaire: Paetzold, Michael, 88310 Grimaud (FR)
(72) Inventeur: Paetzold, Michael, 88310 Grimaud (FR)
(74) Mandataire: Fantin, Laurent
(86) Numéro de dépôt international: PCT/FR2014/050664
(87) Numéro de publication internationale: WO 2015/140419

(56) Documents cités:
- CN-A- 103 525 610
- US-A1- 2009 092 695
- PIERMATTEI B ET AL: "Studi preliminari sull'impiego di raspl essiccali nella vinlflcazione in rosso - [Preliminary studies on the use of dried grape stems in red winemaking]", RIVISTA DI VITICOLTURA DI ENOLOGIA, SCARPIS, TREVISO, IT, vol. 52, no. 4, 1 janvier 1999 (1999-01-01), pages 23-31, XP008171995, ISSN: 0370-7865
- Becca: "Grape Stem Extracts: An Example of Utilizing Winery Waste for a More Sustainable Industry", Environmental Science , 5 novembre 2012 (2012-11-05), XP002732805, Extrait de l'Internet: URL:http://www.academicwino.com/2012/11/gr ape-stem-extracts-sustainability.html/ [extrait le 2014-11-20]
- SIERRA RAYNE, ERKAN KARACABEY, G. MAZZA: "Grape cane waste as a source of trans-resveratrol andtrans-viniferin: High-value phytochemicals withmedicinal and anti-phytopathogenic applications", INDUSTRIAL CROPS AND PRODUCTS, [Online] vol. 27, 2008, pages 335-340, XP002732806, Extrait de l'Internet: URL:http://ac.els-cdn.com/S092666900700166 5/1-s2.0-S0926669007001665-main.pdf?_tid=a 77280d0-717e-11e4-8de4-00000aab0f6c&acdnat =1416575131_2ea6e64b238e8c053eec53d079fb61 21> [extrait le 2014-11-21]
- MARIA ANASTASIADI, HARRIS PRATSINIS, DIMITRIS KLETSAS, ALEXIOS-LEANDROS SKALTSOUNIS, SERKOS A. HAROUTOUNIAN: "Grape stem extracts: Polyphenolic content and assessment of their in vitroantioxidant properties", LWT - FOOD SCIENCE AND TECHNOLOGY, [Online] vol. 48, 2012, pages 316-322, XP002732807, DOI: 10.1016/j.lwt.2012.04.006 Extrait de l'Internet: URL:http://ac.els-cdn.com/S002364381200176 4/1-s2.0-S0023643812001764-main.pdf?_tid=4 a4f8b6c-7180-11e4-8857-00000aab0f27&acdnat =1416575834_9c843d181da239b868239c0f6ff10a 79> [extrait le 2014-11-21]
- LAN PING, NICOLAS BROSSE, POULOMI SANNIGRAHI, ARTHUR RAGAUSKAS: "Evaluation of grape stalks as a bioresource", INDUSTRIAL CROPS AND PRODUCTS, [Online] vol. 33, 2011, pages 200-204, XP002732808, DOI: 10.1016/j.indcrop.2010.10.009 Extrait de l'Internet: URL:http://ac.els-cdn.com/S092666901000254 2/1-s2.0-S0926669010002542-main.pdf?_tid=4 e309dbe-7182-11e4-bf8f-00000aab0f27&acdnat =1416576700_4ad02672dc1d6b17aa88cabe381a0f d1> [extrait le 2014-11-21]
- EDER C. LIMA ET AL.: "Adsorption of Cu(II) on Araucaria angustifolia wastes: Determinationof the optimal conditions by statistic design of experiments", JOURNAL OF HAZARDOUS MATERIALS, [Online] vol. 140, 2007, pages 211-220, XP002732809, DOI: 10.1016/j.jhazmat.2006.06.073 Extrait de l'Internet: URL:http://ac.els-cdn.com/S030438940600725 4/1-s2.0-S0304389406007254-main.pdf?_tid=c 9cc277e-7185-11e4-8b7c-00000aacb35d&acdnat =1416578195_175fa7337ce1c8a8101e55d97e91fc c8> [extrait le 2014-11-21]

## Description

La présente invention se rapporte à une utilisation de rafles lignifiées d'au moins un extrait de rafles lignifiées dans une composition cosmétique, un complément alimentaire, un produit nutritionnel, un aliment ou une boisson. L'invention se rapporte également à des rafles lignifiées broyées, un extrait de rafles lignifiées ou à une composition en contenant. Dans le cas d'un vin rouge, une méthode de vinification comprend les étapes suivantes :
La vendange plus ou moins égrappée et écrasée est introduite dans une cuve de fermentation. L'opération d'égrappage consiste à écarter les tiges et les rafles en excès. Ces parties ligneuses provoquent généralement des goûts herbacés lors des cuvaisons longues. Pendant la cuvaison, une fermentation alcoolique et une extraction s'opèrent. Durant cette phase, les matières solides (pulpe, peau,..) remontent en surface et flottent sur le jus. Cet amas de matières solides est appelé chapeau. Régulièrement, le jus est pompé en fond de cuve pour être répandu sur le chapeau afin de favoriser l'extraction. A l'issue de la phase de fermentation alcoolique, on procède à l'écoulage de la cuve afin de récupérer le jus, appelé « vin de goutte ». Les matières solides restées en fond de cuve sont appelées le marc. Ce dernier est sorti de la cuve pour être pressé de manière à obtenir le vin de presse. Le restant du marc sec et compacté est définitivement séparé du vin. Il peut être utilisé pour faire du compostage.

En suivant, le vin peut être soumis à une phase de fermentation malolactique.

A l'issue de cette dernière fermentation, le vin est introduit dans des fûts pour y être élevé pendant plusieurs mois avant d'être embouteillé. Pendant cette période, les fines particules en suspension dans le vin se déposent doucement au fond des fûts pour former une lie. Seule, la partie liquide (haut du fût) sera mise en bouteille.

Le vin de goutte et le vin de presse sont généralement élevés de manière séparée et pourront être assemblés ultérieurement en fonction des propriétés organoleptiques de chacun.

Lorsque la structure du vin le justifie, l'élevage du vin est opéré dans des fûts en bois afin de compléter la structure du vin par l'apport de tannins externes issus du bois du fût et de faire évoluer ses arômes.

Selon d'autres techniques d'élevage du vin, ce dernier peut être stocké dans des cuves inertes dans lesquelles sont introduits des copeaux de bois. En variante, les copeaux de bois peuvent être introduits lors de la vinification, par exemple lors de la fermentation alcoolique.

L'apport du bois (fûts ou copeaux) dépend de l'essence du bois et de la chauffe subie par le bois.

Le bois utilisé étant généralement du chêne, les apports aromatique et gustatif sont de ce fait étroitement liés à la chauffe du bois.

Enfin, la technique d'assemblage permet de mélanger plusieurs cuvées provenant par exemple de différentes parcelles d'une propriété, du vin de goutte ou du vin de presse afin d'élaborer un vin.

Selon d'autres techniques décrites dans des publications, après la phase d'égrappage, il est possible de traiter les rafles « vertes » (ou à l'état végétal) avec de la vapeur de manière à extraire certains composants. Cependant, cette solution n'est pas privilégiée car à l'état végétal, les rafles libèrent des composants qui ont tendance à procurer au vin des goûts herbacés ou d'amertume.

Dans le domaine viticole, les rafles « vertes » et le marc de raisins qui contient la peau et les pépins des grains de raisins sont considérés comme des déchets. Piermattei *et al.* (1999) décrivent l'utilisation de rafles lignifiées dans la fabrication du vin rouge.

Dans le domaine cosmétique, il est connu d'utiliser les pépins ou des peaux des raisins pour en extraire certains composants afin d'obtenir un principe actif.

Cependant, les rafles « vertes » ou à l'état végétal sont considérées comme des déchets, et sont éventuellement valorisées comme amendement organique.

L'invention propose une autre solution pour valoriser les rafles « vertes » ou l'état végétal. A cet effet, l'invention a pour objet l'utilisation de rafles lignifiées, d'au moins un extrait de rafles lignifiées ou d'une composition en contenant dans une composition cosmétique, un complément alimentaire, un produit nutritionnel, un aliment ou une boisson.

Pour la présente demande, on entend par rafle aussi bien la rafle entière que des morceaux de rafles.

Pour la suite de la description, on entend par lignifié un état des rafles dans lequel elles ont pris l'aspect du bois, les rafles étant vertes et ayant l'aspect d'un végétal au moment de la vendange et marron à l'état lignifié. Ainsi, la lignification est un processus par lequel les membranes de certaines cellules végétales se transforment, sous l'action de la lignine, en bois.

Ainsi, selon l'invention, les rafles subissent une phase de lignification pour retirer les goûts herbacés et d'amertume avant d'être introduites dans le vin.

Avantageusement, les rafles sont séchées pour accélérer la lignification. Ce séchage peut être réalisé naturellement ou artificiellement. De manière naturelle, le séchage dure de l'ordre de 3 à 5 semaines pour obtenir la lignification des rafles.

A l'issue de la vendange, la rafle comprend un pédoncule, des pédicelles et de petits renflements appelés pinceau situés à l'extrémité des pédicelles au niveau desquels sont fixés les grains.

Avantageusement, après la phase de lignification, les rafles subissent une étape de tri visant à retirer les petits renflements ou pinceau qui ont tendance à se détacher du reste de la rafle après le séchage.

De préférence, le tri permet de ne conserver que la partie superficielle des rafles ou des morceaux de rafles. Par partie superficielle, on entend la peau, l'écorce ou la partie ligneuse des rafles si ces dernières ne sont que partiellement lignifiées.

Selon une première variante, après la phase de lignification et à l'issue de l'éventuelle opération de triage, les rafles sont découpées en morceaux homogènes.

Selon un mode de réalisation préféré, les rafles lignifiées et de préférence triées et découpées sont soumises à une étape de chauffe à la manière du bois d'une barrique. A cet effet, les rafles lignifiées sont torréfiées ou toastées. Cette étape permet de pouvoir moduler les propriétés olfactive et gustative des rafles en fonction de la température et de la durée de la chauffe.

Selon une autre variante, après la phase de lignification et après l'éventuelle opération de triage, les rafles lignifiées sont broyées. Avantageusement, la granulométrie de la poudre de rafles lignifiées obtenue après broyage doit être inférieure à 0,5 mm.

Par rafles lignifiées en poudre, on entend le broyat obtenu par le broyage des rafles lignifiées ou une fraction du broyat.

Cette variante permet d'obtenir une meilleure extraction des composants provenant des rafles lignifiées.

Préalablement à l'étape de broyage ou éventuellement ultérieurement, les rafles lignifiées broyées ou non sont soumises à une étape de chauffe à la manière du bois d'une barrique. A cet effet, les rafles lignifiées broyées ou non sont torréfiées ou toastées. Cette étape permet de pouvoir moduler les propriétés olfactive et gustative des rafles en fonction de la température et de la durée de la chauffe.

L'invention propose également un dispositif pour produire des rafles lignifiées. Ce dispositif comprend un four de préférence continu. Ainsi, les rafles avantageusement découpées et triées défilent sous des moyens de chauffage.

Selon un mode de réalisation, le dispositif comprend un poste assurant la découpe des rafles et un autre pour trier les rafles découpées et retirer les petits renflements situés à l'extrémité des pédicelles par exemple un tamis. A l'issue de ce tri, les rafles découpées et triées sont disposées sur un convoyeur qui traverse un four. Le four peut également assurer la torréfaction des rafles découpées et triées.

L'invention propose également un produit ou une composition comprenant des rafles lignifiées entières, en morceaux ou en poudre.

Selon un mode de conditionnement, le produit contient exclusivement des rafles lignifiées entières, en morceau ou en poudre.

Selon un autre mode de conditionnement, le produit comprend une phase aqueuse contenant des rafles lignifiées entières, en morceaux ou en poudre.

Selon un autre mode de conditionnement, les rafles lignifiées entières, en morceaux ou en poudre sont conditionnées sèches dans un contenant perméable. Dans ce cas, l'invention propose un produit comprenant un contenant perméable qui contient des rafles lignifiées entières, en morceaux ou en poudre.

Par contenant perméable, on entend qu'au moins une portion de la paroi du contenant est perméable au liquide tout en retenant les rafles lignifiées entières, en morceaux ou en poudre. Cette solution permet de pouvoir retirer tous les éléments des rafles lignifiées introduits dans un liquide à l'issue d'une phase d'extraction, de macération, d'infusion. Selon un mode de réalisation, le contenant est un sachet, comme un sachet à infusion par exemple.

L'invention propose également un extrait de rafles lignifiées ou une composition comprenant au moins un extrait de rafles lignifiées. L'extrait de rafles lignifiées peut être obtenu à partir de rafles lignifiées, éventuellement triées et/ou torréfiées et/ou toastées. Selon une première variante, l'extrait de rafles lignifiées est obtenu par un procédé comprenant au moins une étape d'extraction aqueuse.

De préférence, le procédé d'extraction aqueuse comprend au moins une étape de solubilisation de rafles lignifiées dans l'eau ou dans un mélange d'eau et d'alcool. Avantageusement, le procédé d'extraction aqueuse comprend au moins une étape d'infusion.

De manière simplifiée, l'extraction aqueuse peut consister à faire macérer dans un liquide des rafles lignifiées entières, en morceaux ou en poudre, les rafles lignifiées entières, en morceaux ou en poudre étant retirées du liquide après une certaine durée.

Selon une deuxième variante, l'extrait de rafles lignifiées est obtenu par un procédé comprenant au moins une étape d'hydrolyse de rafles lignifiées. Cette hydrolyse peut être une hydrolyse acide ou basique ou une hydrolyse enzymatique.

Les produits précédemment cités à base de rafles lignifiées sont riches en polyphénols, notamment en résveratrol, en mélatonine, en tanins, en arômes, ....

Les produits précédemment cités à base de rafles lignifiées auront des caractéristiques organoleptiques qui seront différentes en fonction du cépage dont sont issues les rafles. Selon une première utilisation, les rafles lignifiées sont utilisées dans une méthode de vinification ou d'élevage d'un vin.

Selon un premier mode opératoire, la méthode de vinification ou d'élevage comprend une étape qui consiste à faire macérer dans le vin des rafles lignifiées entières, en morceaux ou en poudre.

La macération des rafles peut être réalisée indifféremment lors des différentes phases de l'évolution du vin, par exemple dès l'introduction de la vendange en cuve.

De préférence, l'ajout des rafles lignifiées est réalisé à l'issue des phases de fermentation lors de la phase d'élevage du vin. La macération des rafles lignifiées peut être contrôlée de manière plus précise lors de cette phase.

Selon une première variante, après l'opération d'égrappage ou de pressurage, les rafles sont conservées. Elles sont séchées naturellement de manière à obtenir la lignification des rafles. Elles sont de préférence utilisées pour les vendanges suivantes. Ainsi, selon ce mode opératoire, les rafles lignifiées introduites dans le vin ne sont pas celles qui supportaient les grains de raisin dont est issu le vin.

Selon une autre variante, après l'opération d'égrappage ou de pressurage, les rafles sont lignifiées en séchant naturellement. Après une durée de l'ordre de 1 à 5 semaines, elles sont introduites dans le vin issu des grains de raisin portés par les rafles lignifiées.

Selon une autre variante, après l'opération d'égrappage ou de pressurage, les rafles sont lignifiées à l'aide d'un four qui peut également assurer la torréfaction. Ainsi, selon ce mode opératoire, les rafles lignifiées introduites dans le vin sont celles qui supportaient les grains de raisin dont est issu le vin.

Selon une caractéristique de l'invention, le dosage du produit ou de la composition comprenant des rafles lignifiées entières, en morceaux ou en poudre introduit(e) dans le vin est compris entre 1 gr/hl et 10 gr/hl.

La durée de macération des rafles lignifiées ainsi que leurs quantités sont ajustées en fonction des propriétés olfactive et gustative recherchées.

Selon un deuxième mode opératoire, la méthode de vinification et d'élevage d'un vin comprend une étape qui consiste à introduire dans le vin un produit ou une composition comprenant de la poudre de rafles lignifiées, un extrait de rafles lignifiées ou une phase aqueuse dans laquelle des rafles lignifiées entières, en morceaux ou en poudre ont macérées ou infusées.

Les avantages de la méthode de vinification visant à faire macérer des rafles lignifiées dans le vin sont les suivants :
En premier lieu, cette solution permet de valoriser un constituant de la vendange à savoir les rafles dont les caractéristiques olfactive et gustative n'étaient pas exploitées. A contrario, les rafles non lignifiées avaient tendance à être retirées lors de l'égrappage afin de ne pas conférer au vin des goûts herbacés ou d'amertume.

En second lieu, il permet un apport avec des nuances olfactive et gustative jusqu'ici non exploitées qui complètent le panel des propriétés organoleptiques d'un vin. Ainsi, il est possible d'ajuster l'apport en fonction de la nature du cépage et du terroir des rafles, en fonction du séchage des rafles, en fonction de la chauffe des rafles.

Les rafles lignifiées peuvent être utilisées pour d'autres applications.

Selon une autre application, l'invention propose l'utilisation de rafles lignifiées entières, en morceaux ou en poudre, d'au moins un extrait de rafles lignifiées ou d'une composition en contenant comme principe actif dans une composition cosmétique. Les compositions cosmétiques ainsi obtenues, riches en polyphénols, sont plus particulièrement destinées au soin de la peau.

Selon une autre application, l'invention propose l'utilisation de rafles lignifiées entières, en morceaux ou en poudre, d'au moins un extrait de rafles lignifiées ou d'une composition en contenant comme principe actif nutritionnel dans un complément alimentaire ou un produit nutritionnel.

Selon une autre application, l'invention propose l'utilisation de rafles lignifiées entières, en morceaux ou en poudre, d'au moins un extrait de rafles lignifiées ou d'une composition en contenant comme exhausteur, agent de saveur ou agent aromatisant dans des boissons ou des aliments.

Ainsi, il est possible d'aromatiser une bière avec un extrait de rafles lignifiées. Pour cette boisson, l'extrait de rafles lignifiées est utilisé pour ses tanins, ses aromes, sa sucrosité et en tant qu'exhausteur. A titre d'exemple, la bière est aromatisée au merlot, en ajoutant à la bière un extrait de rafles lignifiées issues de grappes de merlot.

Il est également possible d'obtenir une boisson sans alcool ayant des arômes et un goût proches de ceux du vin en incorporant dans une boisson sans alcool un extrait de rafles lignifiées.

## Revendications

1. Utilisation de rafles lignifiées entières, en morceaux ou en poudre, d'au moins un extrait de rafles lignifiées ou d'une composition en contenant comme exhausteur, agent de saveur ou agent aromatisant dans des boissons ou des aliments, les rafles étant préalablement lignifiées pendant une durée de 1 à 5 semaines afin que des membranes de certaines cellules végétales des rafles se transforment, sous l'action de la lignine, en bois.

2. Utilisation de rafles lignifiées, d'au moins un extrait de rafles lignifiées ou d'une composition en contenant selon la revendication 1, **caractérisée en ce que** les rafles lignifiées sont broyées.

3. Rafles lignifiées **caractérisées en ce qu'**elles sont préalablement séchées pendant une durée de 1 à 5 semaines afin que des membranes de certaines cellules végétales des rafles se transforment, sous l'action de la lignine, en bois puis broyées.

4. Extrait de rafles lignifiées **caractérisé en ce qu'**il est obtenu par un procédé comprenant une étape de séchage pendant une durée de 1 à 5 semaines afin que des membranes de certaines cellules végétales des rafles se transforment, sous l'action de la lignine, en bois et au moins une étape d'extraction aqueuse.

5. Extrait de rafles lignifiées selon la revendication 4, **caractérisé en ce que** le procédé comprend au moins une étape de solubilisation de rafles lignifiées dans l'eau.

6. Extrait de rafles lignifiées selon la revendication 4, **caractérisé en ce que** le procédé comprend au moins une étape de solubilisation de rafles lignifiées dans un mélange d'eau et d'alcool.

7. Extrait de rafles lignifiées selon l'une des revendications 4 à 6, **caractérisé en ce que** le procédé comprend au moins une étape d'infusion de rafles lignifiées.

8. Extrait de rafles lignifiées **caractérisé en ce qu'**il est obtenu par un procédé comprenant une étape de séchage pendant une durée de 1 à 5 semaines afin que des membranes de certaines cellules végétales des rafles se transforment, sous l'action de la lignine, en bois et au moins une étape d'hydrolyse de rafles lignifiées.

9. Extrait de rafles lignifiées selon la revendication 8, **caractérisé en ce que** l'hydrolyse est une hydrolyse acide ou basique.

10. Extrait de rafles lignifiées selon la revendication 8, **caractérisé en ce que** l'hydrolyse est une hydrolyse enzymatique.

11. Composition comprenant un extrait de rafles lignifiées selon l'une des revendications 4 à 10.

## Patentansprüche

1. Verwendung von ganzen verholzten Rappen in Stücken oder in Pulverform, wenigstens eines Extrakts aus verholzten Rappen oder einer Zusammensetzung, die diese als Geschmacksverstärker, Geschmacksstoff oder Aromastoff enthält, in Getränken oder Lebensmitteln, wobei die Rappen zuvor während einer Dauer von 1 bis 5 Wochen verholzt werden, damit sich die Membranen bestimmter Pflanzenzellen der Rappen unter der Einwirkung von Lignin in Holz umwandeln.

2. Verwendung der verholzten Rappen, wenigstens eines Extrakts aus verholzten Rappen oder einer Zusammensetzung, die diese enthält, nach Anspruch 1, **dadurch gekennzeichnet, dass** die verholzten Rappen zerkleinert sind.

3. Verholzte Rappen, **dadurch gekennzeichnet, dass** diese zuvor während einer Dauer von 1 bis 5 Wochen getrocknet werden, damit die Membranen von bestimmten Pflanzenzellen der Rappen sich unter der Einwirkung von Lignin in Holz umwandeln, und dass diese dann zerkleinert werden.

4. Extrakt aus verholzten Rappen, **dadurch gekennzeichnet, dass** diese durch ein Verfahren erhalten werden, das einen Verfahrensschritt der Trocknung für eine Dauer von 1 bis 5 Wochen aufweist, damit die Membranen von bestimmten Pflanzenzellen der Rappen sich unter der Einwirkung von Lignin in Holz umwandeln, und das wenigstens einen Verfahrensschritt einer wässrigen Extraktion aufweist.

5. Extrakt aus verholzten Rappen nach Anspruch 4, **dadurch gekennzeichnet, dass** das Verfahren wenigstens einen Verfahrensschritt der Solubilisierung der verholzten Rappen in Wasser umfasst.

6. Extrakt aus verholzten Rappen nach Anspruch 4, **dadurch gekennzeichnet, dass** das Verfahren wenigstens einen Verfahrensschritt der Solubilisierung von verholzten Rappen in einer Mischung von Wasser und Alkohol umfasst.

7. Extrakt aus verholzten Rappen nach einem der Ansprüche 4 bis 6, **dadurch gekennzeichnet, dass** das Verfahren wenigstens einen Verfahrensschritt eines Aufgusses der verholzten Rappen umfasst.

8. Extrakt aus verholzten Rappen, **dadurch gekennzeichnet, dass** dieser durch ein Verfahren erhalten wird, das einen Verfahrensschritt der Trocknung während einer Dauer von 1 bis 5 Wochen umfasst, damit die Membranen von bestimmten Pflanzenzellen der Rappen sich unter der Einwirkung von Lignin in Holz umwandeln, und das wenigstens einen Verfahrensschritt der Hydrolyse der verholzten Rappen umfasst.

9. Extrakt aus verholzten Rappen nach Anspruch 8, **dadurch gekennzeichnet, dass** die Hydrolyse eine saure oder basische Hydrolyse ist.

10. Extrakt aus verholzten Rappen nach Anspruch 8, **dadurch gekennzeichnet, dass** die Hydrolyse eine enzymatische Hydrolyse ist.

11. Zusammensetzung mit einem Extrakt aus verholzten Rappen nach einem der Ansprüche 4 bis 10.

## Claims

1. Use of lignified stalks that are whole, in pieces, or in powder form of at least one lignified stalk extract or of a composition containing it as an enhancer, a flavoring agent or an aromatizing agent in beverages or food, the stalks being lignified beforehand for a period of 1 to 5 weeks so that the membranes of certain vegetable cells of the stalks are transformed, under the action of lignin, into wood.

2. Use of lignified stalks, of at least one lignified stalk extract or of a composition containing it according to claim 1, **characterized in that** the lignified stalks are ground.

3. Lignified stalks **characterized in that** they are dried beforehand for a period of 1 to 5 weeks so that the membranes of certain vegetable cells of the stalks are transformed, under the action of lignin, into wood.

4. Lignified stalk extract **characterized in that** it is obtained by a method that comprises a drying step for a period of 1 to 5 weeks so that the membranes of certain vegetable cells of the stalks are transformed, under the action of lignin, into wood.

5. Lignified stalk extract according to claim 4 wherein the method comprises at least one step for solubilization of lignified stalks in water.

6. Lignified stalk extract according to claim 4, wherein the method comprises at least one step for solubilization of lignified stalks in a mixture of water and alcohol.

7. Lignified stalk extract according to one of the claims 4 to 6, wherein the method comprises at least one step for infusion of lignified stalks.

8. Lignified stalk extract **characterized in that** it is obtained by a method that comprises a drying step for a period of 1 to 5 weeks so that the membranes of certain vegetable cells of the stalks are transformed, under the action of lignin, into wood and at least one hydrolysis step of lignified stalks.

9. Lignified stalk extract according to Claim 8, wherein the hydrolysis is an acid or base hydrolysis.

10. Lignified stalk extract according to Claim 8, wherein the hydrolysis is an enzymatic hydrolysis.

11. Composition comprising a lignified stalk extract according to one of claims 4 to 10.
